# EUROPEAN PATENT APPLICATION

(11) **EP 2 702 886 A1**
(43) Date of publication of application: **05.03.2014**
(21) Application number: 12425145.5
(22) Date of filing: 31.08.2012
(51) Int. Cl.: A42B 3/04, G02B 27/01, A61B 1/06, A61B 19/00, G02B 21/00, G02B 23/12

(54) **Device suitable for mounting and orienting a video camera on head mounted binoculars**

(71) Applicant: Auxilium Opera Di Fabio Peruzzi & C. snc., 00177 Roma (IT)
(72) Inventor: Peruzzi, Fabrizio, 00177 Roma (IT); Peruzzi, Fabio, 00177 Roma (IT)

(57) **Abstract**

A device suitable for mounting and orienting a video camera on head mounted binoculars, comprises binoculars (b) having two telescopes rotating around a central body that is provided with a rear part and a front part. The central body of the binoculars (b) is supported in its rear part to a headband (f) of a user by means of a first support element (1) tiltable with respect to the same headband (f). The central body of the binoculars (b) supports in its front part a video camera (T) by a second support element (2).

## Description

The present invention relates to a device suitable for mounting and orienting a video camera on head mounted binoculars. Said device is useful for making an integrated system for recording or transmitting videos and images of surgical interventions, in particular dental surgery, and also of industrial technological phases.

It is well known how to mount a video camera on a helmet by means of brackets in various sports sectors such as car and motorcycle racing, hiking and other, where the main requirement is to permit a user to record what he/she sees keeping his/her hands free to operate.

Said invention addresses in particular a combination of binoculars and video camera where it is required to align the video camera with the axis of symmetry of binoculars and make coincident their planes of focus.

The patent U.S. 4,621,283 describes a head-mounted imaging system which employs a headband to be worn on the head of a surgeon during a surgical procedure. A video camera is mounted on the headband which also provides a light beam that is reflected by a mirror to be directed to an object to be observed. The surgeon wears a magnifying device such as binoculars facing the same object to be observed. It is understood that the video camera is mounted separately from the binoculars and this implies difficulties in centring the video camera exactly and constantly on the object to be observed. The patent U.S. 4,797,736 discloses a head mounted apparatus for illuminating a work site and for transmitting a visual image of the work site to a remote location for viewing on a television screen. For this purpose a video camera and a light source are mounted on a headband which is worn by the user. The video camera lens is generally disposed between the user's eyes to allow shooting the same image as the user is seeing. The light source and the video camera are independently pivotally mounted on a bracket, and the bracket is pivotally mounted to the headband. A series of three pivotally attachments is provided so that the light source, the video camera and the bracket must be individually adjusted to obtain the right orientation. Furthermore, the described arrangement does not disclose how to use binoculars adapted to enlarge the object to be observed.

The international patent application PCT/SE2010/050469 describes a mounting system for head-worn equipment, comprising a mount for mounting the system to the user's head, an attachment for attachment of the head-worn equipment, a flip joint arranged between the mount and the attachment capable to assume a use position and a flipped-up position, and a tilt joint for adjustment of an angle of the head-worn equipment in the use position. The above mentioned system has no video camera and no light source, and it is intended mainly for night vision.

A main object of the present invention is to provide a device for mounting a video camera on binoculars, the binoculars being supported by a first support element tiltable with respect to a headband, helmet or the like, and the video camera being supported by means of a second support element having a lower portion aligned with the central body of the binoculars.

A further object of the invention is to provide a system with a support for a light source.

Yet another object of the invention is to provide a mechanical stop to limit the angular aperture of the binoculars, allowing the distance of the telescopes of the binoculars to be fixed based on the distance between the eyes of the doctor or other operator.

Yet another object of the invention is to centre the support of the video camera and the light source.

An additional object of the invention is to provide a support that facilitates the locking of the ball joint of the binoculars with improved ergonomics.

In conclusion, the invention provides the mounting of a video camera whose plane of focus is coincident with the focus of the magnifying binoculars, so that the operator instinctively approaches and departs, to focus images of interest, storing them, moment by moment, not only in his/her memory but also by the video camera.

The so made device allows images to be acquired perfectly in sync, both dimensionally and in the focus, with the real vision of the user, allowing also the use of a system of registration of such images, which may constitute record of medical or industrial interventions.

Furthermore, the system allows, through a network connection, images to be transmitted to receiving devices, such as personal computers, laptops, monitors, televisions, etc., enabling other people involved in the event to monitor user action, on-site or in remote locations. This last feature makes the invention an interesting instrument for those who want, in addition to monitoring the operation that is performed, to use the event for educational purposes, thus avoiding people gathering around the user.

The present invention will be now described, by way of an illustrative but not limiting example with reference to the accompanying drawings, in which:
Figure 1 is a perspective view of a device suitable for mounting and orienting a video camera on head mounted binoculars, the video camera and binoculars being shown with dashed lines, generally comprising a first supporting element for the binoculars to a headband, which is shown only in part, and a second support element for the video camera on which a light source is mounted by a third support element;
Figure 2 shows a front view of the second support element for the video camera on the binoculars in Figure 1;
Figure 3 shows a cross-section view along lines A-A in Figure 2;
Figure 4 is an exploded perspective view of the second support element in Figure 2;
Figure 5 is an exploded perspective view of the third support element in Figure 2;
Figure 5a shows a cross-section made in Figure 5 by a vertical plane, like in Figure 3;
Figure 6 is an exploded perspective view of the first support element in Figure 1;
Figure 7 shows a front view of the first support element for the binoculars in Figure 1;
Figure 8 shows a cross-section view along lines B-B in Figure 7;
Figure 9 shows a cross-section view along lines C-C in Figure 7;
Figure 10 shows a cross-section view along lines D-D in Figure 7; and
Figure 11 and 12 show enlarged details that are circled with 'E' and 'F' in Figure 11.

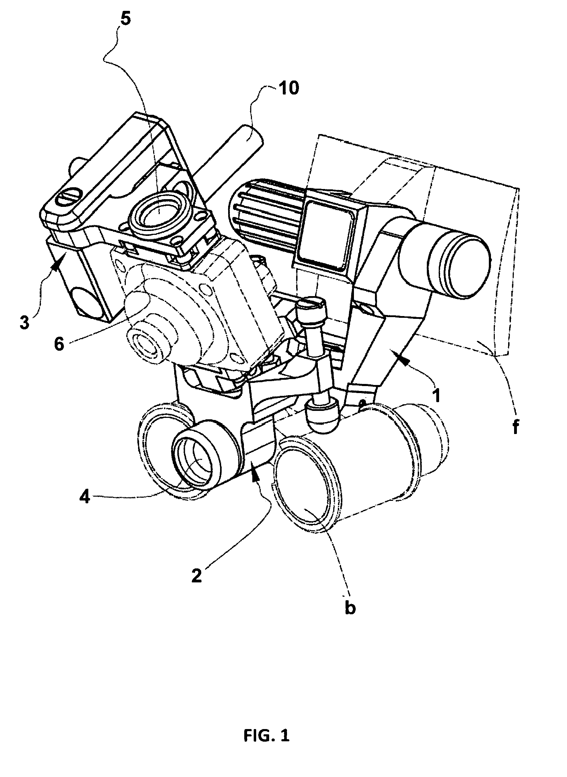

Reference is made initially to Figure 1 wherein a perspective view of the device suitable for mounting and orienting a video camera on head mounted binoculars according to the present invention is shown. There is indicated as T a video camera particularly reduced in size and weight, and binoculars are indicated as b. A first support element 1 supports the binoculars b to a headband f, shown only in part and with a dashed line like the video camera T and the binoculars b. Alternatively, the headband f may be a helmet or the like. A second support element useful for the video camera T is indicated as 2. The second support element 2, as better shown later, is locked on the central cylindrical body between two telescopes of the binoculars b by means of a ring nut 4.

Mounted on the video camera T by a third support element, generally indicated as 3, is a light source 10, such as an optical fibre, shown in part. The third support element 3 is mounted with a ring nut 5 on the video camera T by means of a plate 6, as will be seen in greater detail below.

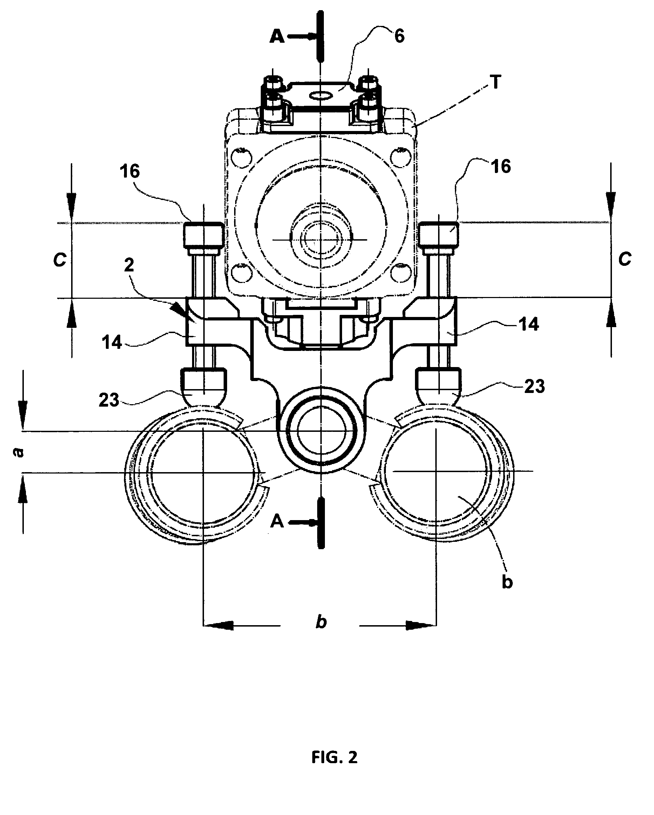

In Figures 2 to 4 the second support element 2 is shown in a front view, in a side cross-section according to lines A-A in Figure 2, and in an exploded perspective view respectively.

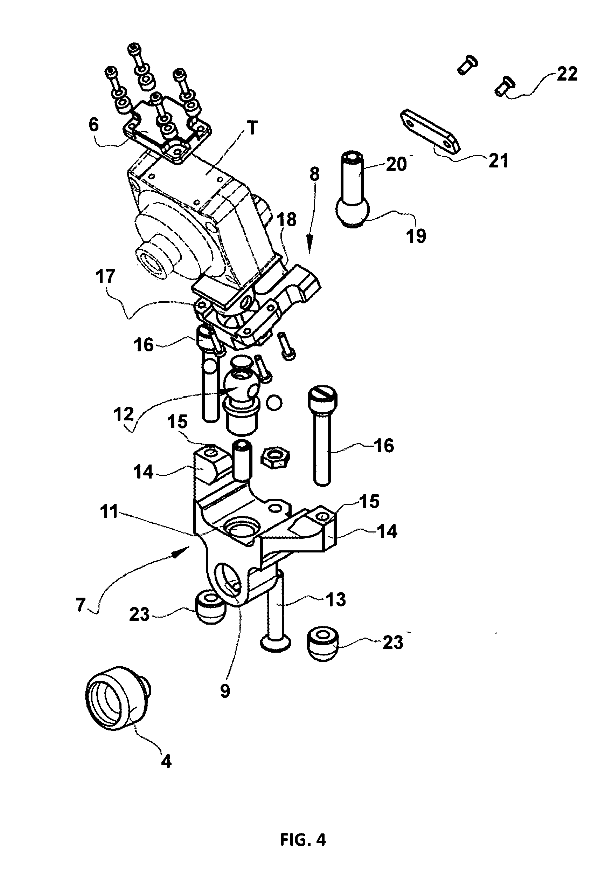

With particular reference to Figure 4, the second support element 2 of the video camera T comprises a V-shaped bottom portion 7 for attachment to the central body of the binoculars b, and an upper portion 8 as a base for the video camera T.

The V-shaped lower portion 7 has a hollow cylindrical body 9 for its insertion on the central body of the binoculars b, where it is locked by the ring nut 4. The V-shaped lower portion 7 also includes a threaded housing 11 made on the top of the hollow cylindrical body 9 and adapted to retain the ball of a ball joint 12, and a rear fixed screw 13 for adjusting the inclination of the video camera T, as will be seen below.

Further, the lower portion 7 has wings 14, 14 diverging from the hollow cylindrical body 9. Each of the wings 14 is provided with a threaded hole 15 for screw coupling with equally threaded rods 16, 16 being adjustably abutted against the cylindrical surface of the telescopes of the binoculars b, as shown in Figure 2.

The upper portion 8 as a base for the video camera T includes a first seat 17 adapted to receive the ball of the ball joint 12 and a partially cylindrical second seat 18, being open at one end for receiving a protuberance 19 of an internally threaded sleeve 20 that is coupled with the rear fixed screw 13 for adjusting the inclination of the video camera T. The second seat 18 is closed at its end by a small bar 21 by screws 22.

The threaded rods 16, 16 coupled with the holes 15, 15 in the wings 14, 14 have contact elements 23, 23 having a spherical tip for resting on the telescopes of the binoculars b, as shown in Figure 2. Indicated as a is the distance between the axis of the central body of the binoculars b coinciding with the axis of rotation of the telescopes, and a plane containing the optical axis of the telescopes. Indicated as b in the same figure 2 is the distance between the eyes of the operator, and as c the position of the two threaded stems 16 that is determined by the mechanical stop to the opening of the binoculars b on the basis of the distance b between the eyes being different for each operator.

The symmetrical positioning of the threaded rods 16, 16 ensures the perfect coaxiality of the video camera T and the coincidence of the imaging field of the video camera T with the plane focused by the binoculars b.

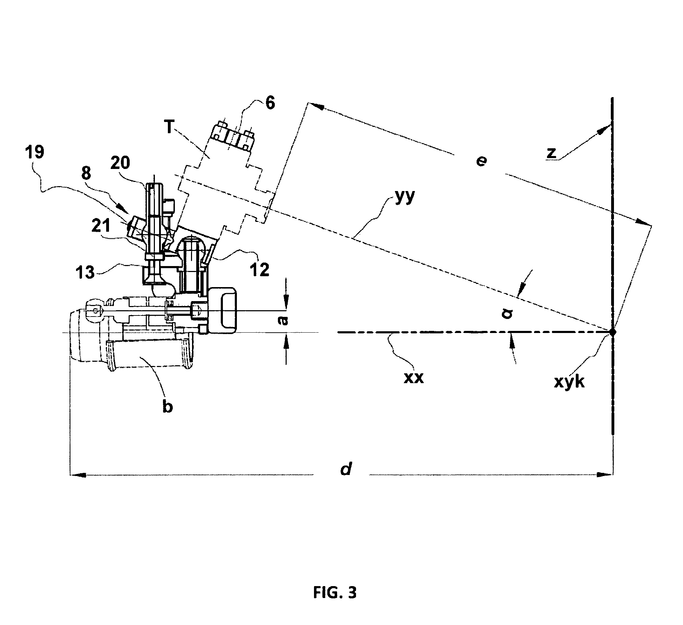

Schematically represented in Figure 3 is the matching point xyk between the axis of symmetry of the telescopes of binoculars b as indicated at xx and the axis of the lens of the video camera T as indicated at yy. This coincidence is obtained by turning the threaded sleeve 20 with respect to the screw 13 locked on the lower portion 7 of the support element 2. The aforesaid mechanical combination sees the protuberance 19 of the threaded sleeve 20 engaging the second seat 18 of the upper portion 8 of the second support element 2 with a mechanical precision coupling. When the threaded sleeve 20 moves along the fixed screw 13, the value of the angle α as an intersection between the symmetry axis xx of the telescopes of the binoculars b and the axis yy of the lens of the video camera T changes. When the threaded sleeve 20 rotates, also the video camera lens gets the due "focus". In fact the distance *e* in Figure 3 represents the distance of focus.

The same operation is performed for positioning the polarised light issued by the optical fibre as a light source.

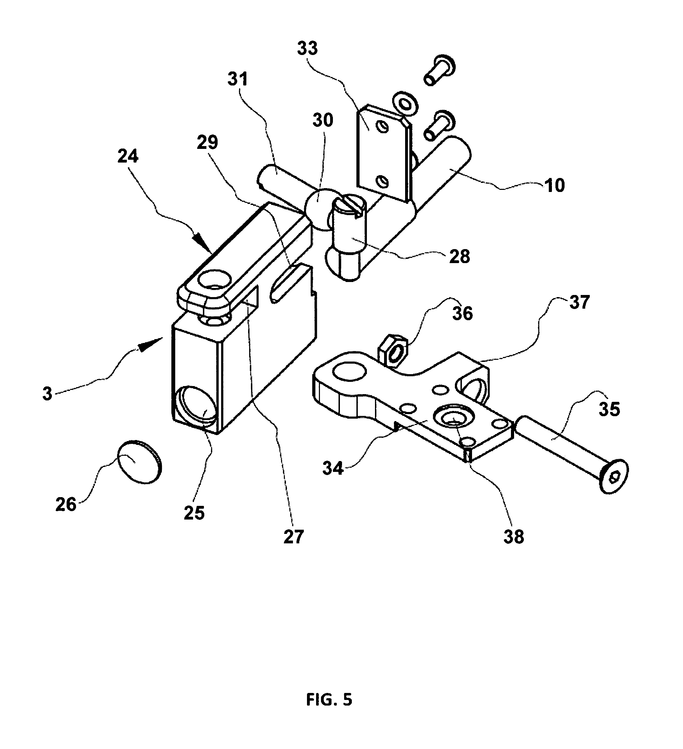

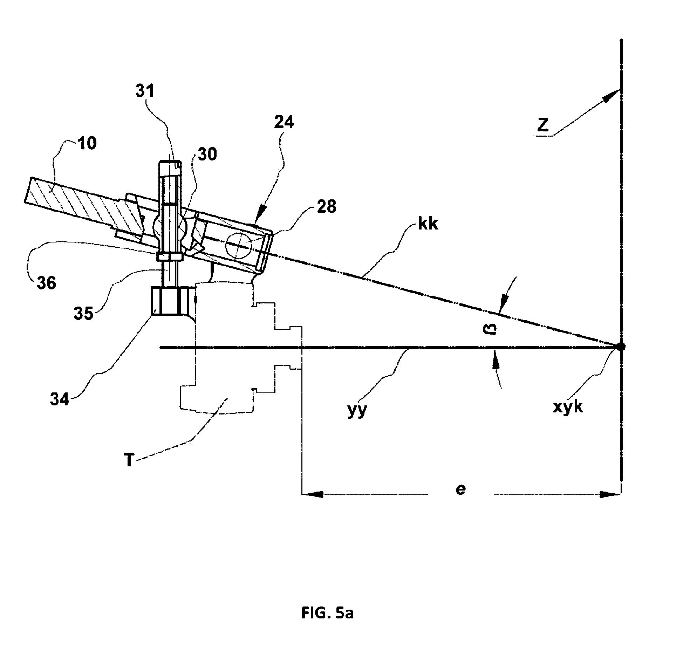

Reference is made in particular to Figure 5 which represents the exploded perspective view of the third support element 3 of the light source with respect to the video camera T, and to Figure 5a, which represents a vertical cross-section in the third supporting element 3 of the light source.

The third support element 3 comprises a support means 24 for the light source such as an optical fibre guide 10 inserted in a hole 25 covered by a lens 26. The support means 24 includes on the top, in a recess 27, a pivot 28 and a cylindrical seat 29 at right angles to the pivot 28 and partially open at one end for receiving a protuberance 30 of an internally threaded sleeve 31 and then closed at its end by a small bar 33 with washers and screws. A first plate 34 is pivotably mounted in the pivot 28. A screw 35 is fixed in a perforated projection 37 of the first plate 34; it is coupled in an adjustable manner with the threaded sleeve 31, which is locked in the operating position by a nut 36 for adjusting the inclination of the support means 3 for the light source. The first plate 34 is equipped with a perforated portion 38 for coupling the first plate 34 by the ring nut 5 to the second plate 6 fixed to the upper wall of the video camera, so as to collimate the light source on the shooting point of the video camera.

Schematically represented in Figure 5a is the matching point xyk between the axis of the lens of the video camera T indicated at yy and the axis of the light source indicated at kk. As described with reference to Figure 3, this coincidence is obtained by turning the threaded sleeve 31 with respect to screw 35 fixed in the projection 37 of the plate 34. The aforesaid mechanical combination sees the protuberance 30 of the threaded sleeve 31 engaging the cylindrical seat 29 of the support means 24 with a mechanical precision coupling. When the threaded sleeve 31 rotates, the value of the angle β changes as an intersection between the axis yy of the lens of the video camera T and the axis kk of the light source. This gives a lighting at a focus distance e.

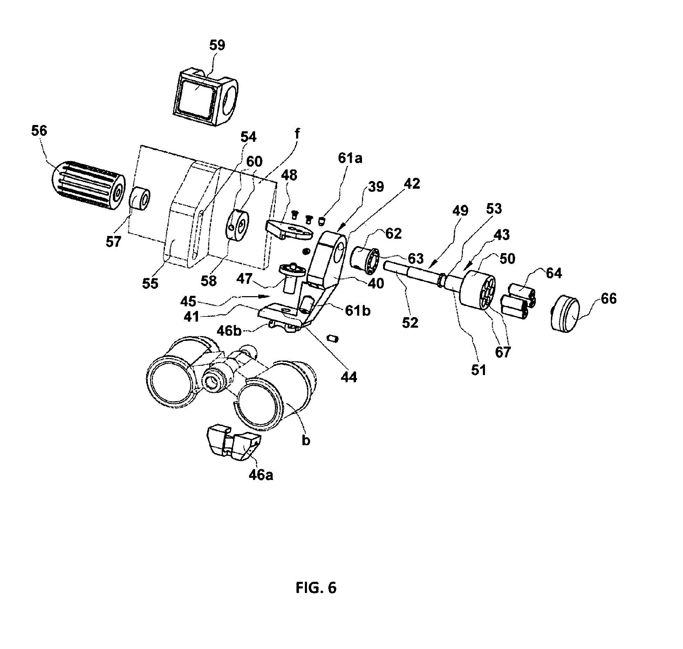

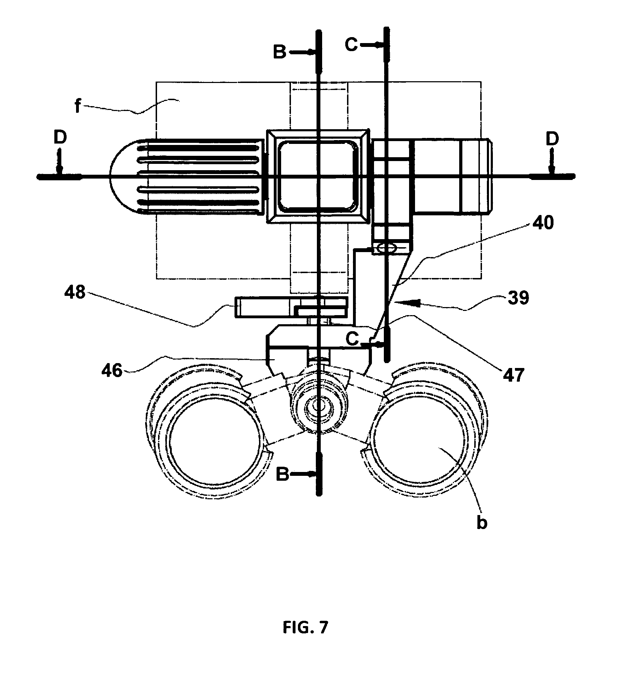

Turning to the description of the first support element 1, Figures 6 to 10, that are an exploded perspective view and a front view of the first support element of Figure 1, and cross-sections along the lines B-B, C-C, and D-D in Figure 7 respectively are taken into consideration.

The first support element 1 of the device suitable for mounting and orienting a video camera on head mounted binoculars according to the present invention comprises a L-shaped support member 39. The L-shaped support element 39 is provided with a core portion 40 and a wing portion 41. The core portion 40 is provided with a through hole 42 having a horizontal axis for a tilting joint, generally indicated as 43, adapted to adjust the position of the binocular b with respect to the headband f. The wing portion 41 is provided with a through hole 44 having a vertical axis for receiving a locking member 45 of the L-shaped support member 39 with the central body of the binoculars b. The locking member 45 comprises a jaw 46a adapted to embrace the back of the central body, the jaw 46 being complementary to a jaw 46b integral with the wing 41 of the L-shaped support member 39. An assembly of the jaws 46a and 46b is indicated as 46 in Figure 7. A locking means 47 rigidly coupled to a control lever 48 locks the central body of the binoculars b to the L-shaped support member 39.

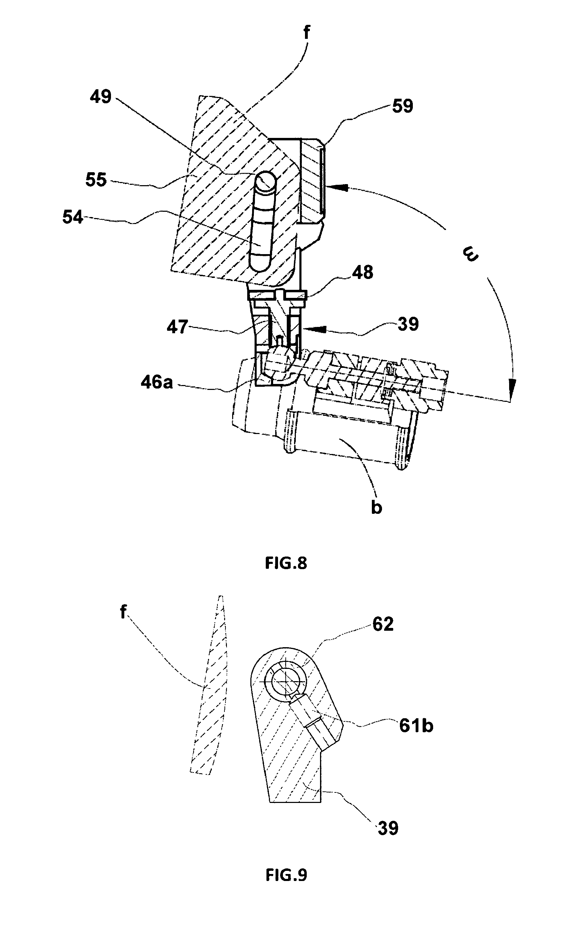

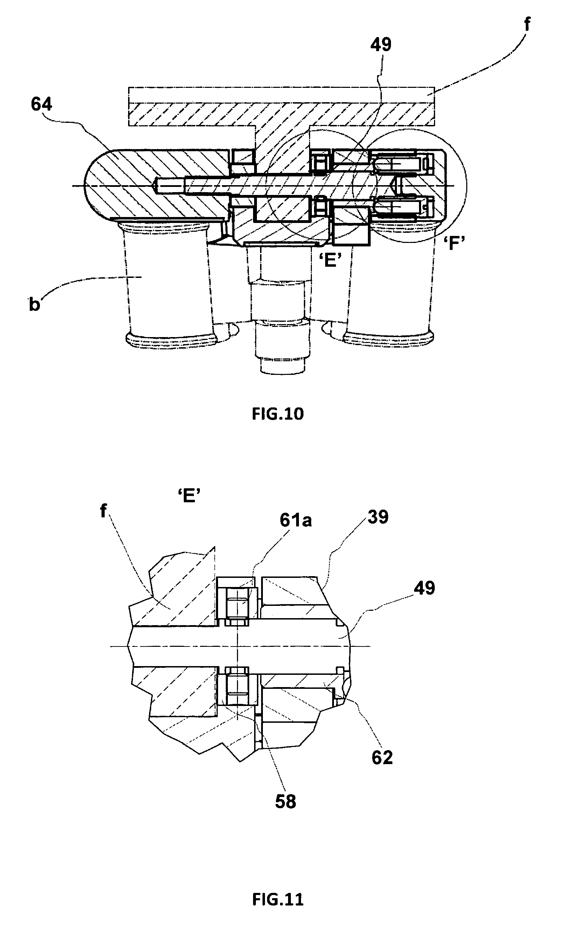

Referring also to Figure 10, the tilting joint 43 includes a shaft 49 having a head 50 and a rod 51, provided with a threaded portion 52 in its end opposite to the head 50, and a circumferential groove 53. As shown in Figure 8 the shaft 49 passes through an opening 54 of a holding element 55 of the headband f. The shaft 49 is retained on the holding element 55 thanks to the that its threaded portion 52 is screwed onto a knob 56 with the aid of a friction disk 57 and of a stop plate 58, both perforated and retained by a logo holder fork 59 in opposite sides of the holding element 55. The stop plate 58 is positioned on the shaft 49 by means of dowels 61a screwed into radial holes 60 of the stop plate 58. The grains 61a hold the stop plate 58 on the shaft 49 at its groove 53. This arrangement is best shown in the detail indicated with 'E' in Figure 10 and enlarged in Figure 11.

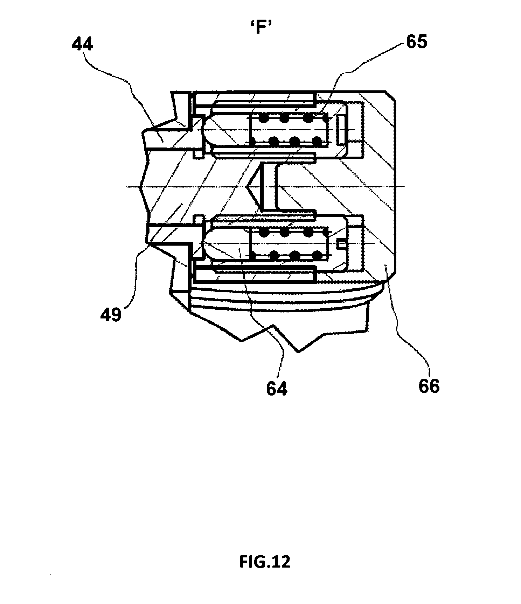

The tilting joint 43 includes a bushing 62 retained in the horizontal through hole 42 of the L-shaped support member 39 by means of a dowel 61b. The bushing 62 is provided frontally with recesses 63. The recesses 63 are designed to receive the ends of pointed pins 64 individually loaded by springs 65 abutted against a cover 66. This arrangement is best shown in the detail indicated by 'F' in Figure 10 and enlarged in Figure 12. The pointed pins 64 and the related springs 65 are housed in coaxial through holes 67 made in the head 50 of the shaft 49. The tilting joint 43 allows the support member 39 to be rotated with respect to the holding element 55 of the headband f for a certain angular displacement (indicated with ω in Figure 8) adjusted by the pointed pins 64 acting on the recesses 63 formed on the bushing 62.

From the above description, it is understood that the L-shaped support member 39 allows inferiorly the central body of the binoculars b to be mounted on the headband f through the locking member 45. Further the knob 56 mounted on the shaft 49 allows the locking in the axial direction of the L-shaped support member 39 and of the shaft 49 same on the headband f. The rotation of the L-shaped support member 39 with respect to the headband f is permitted through the tilting joint 43, that is the L-shaped support member 39 can tilt to the desired angle: this is obtained by means of the pointed pivots 64 that are in engagement with the recesses 63 of the bushing 62, which is integral with the support member 39 through the grain 61b.

In summary, the binoculars b is mounted click rotatable around the shaft 49, and the shaft 49 itself is rigidly fixed to the headband f thanks to the knob 56 screwed on its end and to the bushing 58 connected to the shaft 49 in its groove 53.

It should be apparent that other embodiments of the connection between binoculars b and headband f would be possible without departing from the scope of the invention which is defined in the appended claims.

## Claims

1. A device suitable for mounting and orienting a video camera on head mounted binoculars, comprising binoculars (b) having two telescopes rotating around a central body that is provided with a rear part and a front part, the central body of the binoculars (b) being supported in its rear part to a headband (f) of a user by means of a first support element (1) tiltable with respect to the same headband (f), **characterised in that** said central body of the binoculars (b) supports in its front part a video camera (T) by a second support element (2).

2. The device according to claim 1, wherein the second support element (2) of the video camera (T) comprises a V-shaped lower portion (7) for attachment to the central body of the binoculars (b) and an upper portion (8) as a base for the video camera (T),
the V-shaped lower portion (7) having:
a hollow cylindrical body (9) for its insertion on the central body of the binoculars (b) and the locking thereof by a ring nut (4), a housing (11) formed in the top of the hollow cylindrical body (9) and adapted to retain the ball of a ball joint (12), and a rear fixed screw (13) for adjusting the inclination of the video camera (T), and
divergent wings (14, 14) of the hollow cylindrical body (9), each of the divergent wings (14, 14) being provided with a threaded hole (15) for a screw coupling with corresponding threaded rods (16, 16) adjustably abutting against the cylindrical surface of the telescopes of the binoculars (b);
the upper portion (8) acting as a base for the video camera (T) including a first seat (17) adapted to receive said ball of the ball joint (12) and a second partially cylindrical seat (18) that is open at one end thereof to receive a protuberance (19) of a threaded sleeve (20) coupled to said rear fixed screw (13) for adjusting the inclination, the second partially cylindrical seat (18) being closed at its end by a small bar (21);
so as to collimate the video camera (T) on the object observed by the binoculars (b) by said threaded rods (16, 16) adjustably abutting against the cylindrical surface of the telescopes of the binoculars (b) and on the threaded sleeve (20) coupled to said rear fixed screw (13) for adjusting the inclination of the video camera (T).

3. The device according to claim 1, wherein mounted on the upper wall of the video camera (T) is a light source by a third support element (3) comprising
- a support means for the light source such as an optical fibre guide (10), such a support means including in the top a pivot (28) and a seat (29) perpendicular thereto that is partially cylindrical and open at one end for receiving a protuberance (30) of an internally threaded sleeve (31) and then closed in its end by a small bar (33), the partially cylindrical seat (29) being closed at its end by a small bar (33);
- a first plate (34) pivoted in said pivot (28), a protrusion (37) adapted to receive a screw (35) adjustably coupled with said threaded sleeve (31) fixed with a nut (36) for adjusting the inclination of the support means for the light source, said first plate (34) being provided with a perforated portion for coupling with
- a second plate (6) fixed to the upper wall of the video camera (T) so as to collimate the light source on the shooting point of the video camera (T).

4. The device according to claim 1, wherein the first support element (1) comprises a L-shaped support member (39) having a core portion (40), provided with a horizontal axis through hole (42) for a tilting joint (43) adapted to adjust the position of the binoculars (b) with respect to the headband (f), and a wing portion (41) provided with a vertical axis through hole (44) for receiving a locking means (47) of the L-shaped support member (39) with the central body of the binoculars (b).

5. The device according to claim 4, wherein said tilting joint (43) comprises a shaft (49) in said horizontal axis through hole (42) by a bushing (62) and in a perforated portion (55) of the headband (f), the shaft comprising at one end a head (50) holding pointed pins (64) in order to click adjust the angular position of the support member (39), and at the other end a locking knob (56) of the same shaft in the longitudinal direction by means of a stop plate (58) against said perforated portion (55) of the headband (f).

6. The device according to claim 4, wherein said locking member (45) comprises a jaw (46a) adapted to embrace the back of the central body of the binoculars (b) and a complementary jaw (46b) integral with the wing portion (41) of the L-shaped support member (39), a locking means (47) controlled by a lever (48) being adapted to lock the central body of the binoculars (b) to the L-shaped support member (39).
